# FASCICULE DE BREVET EUROPEEN

(11) **EP 1 076 549 B1**
(45) Date de publication et mention de la délivrance du brevet: **15.09.2004**
(21) Numéro de dépôt: 99918052.4
(22) Date de dépôt: 11.05.1999
(51) Int. Cl.: A61K 7/06

(54) **UTILISATION D'INHIBITEURS DE METALLOPROTEINASES POUR INDUIRE ET/OU STIMULER LA CROISSANCE DES CHEVEUX OU DES POILS ET/OU FREINER LEUR CHUTE**
VERWENDUNG VON METALL-PROTEINASE-INHIBITOREN, UM DAS WACHSTUM DER HAARE ODER KÖRPERHAARE ZU INDUZIEREN UND/ODER ZU STIMULIEREN UND/ODER DEN HAARAUSFALL ZU VERLANGSAMEN
USE OF METALLOPROTINEASE INHIBITORS FOR INDUCING AND/OR STIMULATING GROWTH OF HAIR OR HAIRS AND/OR FOR SLOWING DOWN THEIR LOSS

(30) Priorité: 12.05.1998 FR 9805968
(43) Date de publication de la demande: 21.02.2001
(73) Titulaire: L'OREAL, 75008 Paris (FR)
(72) Inventeur: JARROUSSE, Françoise, F-93190 Livry-Gargan (FR); MAHE, Yann, F-91390 Morsang-sur-Orge (FR)
(74) Mandataire: Galup, Cédric Olivier Nicolas
(86) Numéro de dépôt international: PCT/FR1999/001124
(87) Numéro de publication internationale: WO 1999/058101

(56) Documents cités:
- EP-A- 0 335 554
- EP-A- 0 414 605
- EP-A- 0 455 422
- EP-A- 0 797 978
- WO-A-93/00079
- WO-A-94/06459
- WO-A-96/40185
- GB-A- 2 321 852
- US-A- 4 814 351
- BASE DE DONN ES: "CHEMICAL ABSTRACTS" (SERVEUR: STN); abrégé 115: 141 993, Colombus, OH, USA; & JP 03 074 318 A (K. MIZUMAKI) 28 MARS 1991 XP002091869
- BASE DE DONN ES "CHEMICAL ABSTRACTS" (SERVEUR: STN, Karlsruhe, DE); abrégé 130: 143 951; & JP 11 029 441 A (KANEBO, Ltd) 2 F VRIER 1999 XP002110857
- BASE DE DONN ES "CHEMICAL ABSTRACTS" (SERVEUR: STN, Karlsruhe, DE); abrégé 119: 15 093; & JP 05 043 424 A (SANSEI SEIYAKU KK et al.) 23 F VRIER 1993 XP002110858
- BASE DE DONN ES "CHEMICAL ABSTRACTS" (SERVEUR: STN, Karlsruhe, DE); abrégé 120: 86 083; & JP 05 279 230 A (H. OGAWA et al.) 26 OCTOBRE 1993 XP002110859
- JAROUSSE F. ET AL: 'Identification of clustered cells in human hair follicle responsible for MMP-9 gelatinolytic activity: consequences for the regulation of hair growth' INTERNATIONAL JOURNAL OF DERMATOLOGY vol. 40, pages 385 - 392

## Description

La présente invention concerne l'utilisation dans/ou pour la préparation d'une composition, à titre de principe actif dans un milieu physiologiquement acceptable, d'une quantité efficace d'au moins un inhibiteur tissulaire de métalloprotéinase, ou de tout équivalent biologique fonctionnel, destinée à induire et/ou stimuler la croissance des cheveux ou des poils et/ou freiner leur chute.

Chez l'être humain, la croissance des cheveux et leur renouvellement sont principalement déterminés par l'activité des follicules pileux et de leur environnement dermo-épidermique. Leur activité est cyclique et comporte essentiellement trois phases, à savoir la phase anagène, la phase catagène et la phase télogène.
A la phase anagène active ou phase de croissance, qui dure plusieurs années et au cours de laquelle les cheveux s'allongent, succède une phase catagène très courte et transitoire qui dure quelques semaines, puis une phase de repos, appelée phase télogène, qui dure quelques mois.

A la fin de la période de repos, les cheveux tombent et un autre cycle recommence. La chevelure se renouvelle donc en permanence, et sur les 150000 cheveux environ que comporte une chevelure, à chaque instant, 10% d'entre eux environ sont au repos et seront donc remplacés en quelques mois. Dans un nombre important de cas, la chute précoce des cheveux survient chez des sujets prédisposés génétiquement et elle atteint notamment les hommes. Il s'agit plus particulièrement de l'alopécie androgénétique ou androgénique ou encore androgéno-génétique.

Cette alopécie est essentiellement due à une perturbation du renouvellement capillaire qui entraîne, dans un premier temps, l'accélération de la fréquence des cycles aux dépens de la qualité des cheveux puis de leur quantité. Il se produit un appauvrissement progressif de la chevelure par régression des cheveux dits "terminaux" au stade de duvets. Des zones sont touchées préférentiellement, notamment chez l'homme les golfes temporaux ou frontaux ainsi que l'occiput, tandis que chez les femmes on constate plutôt une alopécie diffuse du vertex.

On recherche depuis de nombreuses années, dans l'industrie cosmétique ou pharmaceutique, des substances permettant de supprimer ou de réduire l'effet de l'alopécie, et notamment d'induire ou de stimuler la croissance des cheveux ou des poils ou de diminuer leur chute.

Dans cette optique, on a certes déjà proposé un grand nombre de composés actifs très divers, comme par exemple le 2,4-diamino-6-piperidinopyrimidine-3-oxyde ou "Minoxidil" décrit dans US 4 596 812 ou encore ses nombreux dérivés comme ceux décrits par exemple dans les demandes de brevet EP 353123, EP 356271, EP 408442, EP 522964, EP 420707, EP 459890, EP 519819.
On peut encore citer la 6-amino-1,2-dihydro-1-hydroxy-2-imino-4-pipéridino pyrimidine et ses dérivés, qui sont décrits. plus particulièrement dans le brevet US-A- 4 139 619.

Il reste, d'une manière générale, qu'il serait intéressant et utile de pouvoir disposer de composés actifs autres que ceux déjà connus.

Or, la demanderesse vient maintenant de découvrir, après d'importantes recherches menées sur la question, qu'un inhibiteur tissulaire de métalloprotéinases, ou tout équivalent biologique fonctionnel, permet d'induire et/ou de stimuler la croissance des cheveux ou des poils, et/ou de diminuer leur chute, de manière efficace, F. Jarrousse et al., Int. J. of Derm, 40, 385-392 (2001).

Les métalloprotéinases (MMPs) sont les membres d'une famille d'enzymes protéolytiques (endoprotéases) qui possèdent un atome de zinc coordonné à 3 résidus cystéine et une méthionine dans leur site actif et qui dégradent les composants macromoléculaires de la matrice extracellulaire et des lames basales à pH neutre (collagène, élastine, etc ...). Très largement répandues dans le monde vivant, ces enzymes sont présentes, mais faiblement exprimées, dans des situations physiologiques normales comme la croissance des organes et le renouvellement des tissus. Leur surexpression chez l'homme et leur activation sont cependant liées à de nombreux processus qui impliquent la destruction et le remodelage de la matrice. Cela entraîne par exemple une résorption non contrôlée de la matrice extracellulaire.

Les métalloprotéinases sont produites et sécrétées sous une forme zymogène inactive (pro-enzyme). Ces formes zymogènes sont ensuite activées dans l'environnement extracellulaire par l'élimination d'une région propeptidique. Les membres de cette famille peuvent s'activer les uns les autres.

La régulation de l'activité des MMPs se produit ainsi au niveau de l'expression des gènes (transcription et traduction), au niveau de l'activation de la forme zymogène, ou au niveau du contrôle local des formes actives.
Les principaux régulateurs de l'activité des MMPs sont les inhibiteurs tissulaires des métalloprotéinases ou TIMPs (tissue inhibitors of metalloproteinases). Cependant, l'expression des MMPs est également modulée par les facteurs de croissance, les cytokines, les produits oncogènes (ras, jun), ou encore les constituants matriciels.

La famille des métalloprotéinases est constituée de plusieurs groupes bien définis basés sur leurs ressemblances en terme de structure et de spécificité de substrat (voir Woessner J. F., Faseb Journal, vol. 5, 1991, 2145). Parmi ces groupes, on peut citer les coliagénases destinées à dégrader les collagènes fibrillaires (MMP-1 ou collagénase interstitielle, MMP-8 ou collagénase de neutrophile, MMP-13 ou collagénase 3), les gélatinases qui dégradent le collagène de type IV ou toute forme de collagène dénaturé (MMP-2 ou gélatinase A (72 kDa), MMP-9 ou gélatinase B (92 kDa) ), les stromyélysines dont le. large spectre d'activité s'adresse aux protéines de la matrice extracellulaire telles que les glycoprotéines (fibronectine, laminine), les protéoglycannes, etc., ou encore les métalloprotéinases membranaires.
La demanderesse a maintenant découvert que les métalloprotéinases sont présentes dans les structures internes des follicules pileux, à savoir dans la gaine épithéliale interne (IRS). Particulièrement, on retrouve la MMP-9 au niveau de l'IRS.

Or, on sait qu'au cours du cycle pilaire le follicule pileux passe d'une localisation basse dans le derme dans la phase anagène a une localisation haute dans le derme lors de la phase télogène. Ce mouvement doit être accompagné d'une modification de la matrice extracellulaire qui permet la migration du follicule, modification qui pourrait être due à une expression des MMPs, entraînant une dégradation contrôlée de ladite matrice extracellulaire. C'est à la fin de la phase télogène qu'intervient la chute du cheveu.Mais on sait aussi que les cytokines et les facteurs de croissance influent sur le cycle pilaire. Par exemple le facteur de croissance épidermique (EGF : epidermal growth factor) favorise *in vitro* la transition depuis la phase anagène vers la phase catagène (formation d'une structure en « club » caractéristique de la phase catagène), phase qui précède la chute des cheveux ou des poils. On sait aussi, comme l'a démontré la demanderesse qu'il existe dans l'alopécie une phase inflammatoire.

La demanderesse a montré que les MMPs et particulièrement la MMP-9, peuvent être induites par l'interleukine-1 et/ou l'EGF en particulier dans les fibroblastes des papilles dermiques.
On comprend donc l'intérêt de diminuer au niveau du cuir chevelu, l'expression des MMPs pour ralentir voir inhiber la dégradation de la matrice périfolliculaire et ainsi ralentir voir stopper la chute des cheveux.

La demanderesse propose donc l'utilisation d'inhibiteurs tissulaires de métalloprotéinases pour induire et/ou stimuler la croissance des cheveux ou des poils et/ou freiner leur chute.

Ainsi, l'invention concerne l'utilisation dans/ou pour la préparation d'une composition, d'une quantité efficace d'au moins un inhibiteur tissulaire de métalloprotéinase ou de tout équivalent biologique fonctionnel, destinée à induire et/ou stimuler la croissance des cheveux ou des poils et/ou freiner leur chute

Par équivalent biologique fonctionnel, on entend toute molécule fonctionnellement équivalente en terme de fonction biologique dont l'un au moins des éléments peut avoir été changé pour un élément équivalent.
A titre d'exemple on peut citer les peptides dont un équivalent biologique peut être un peptide dans lequel au moins un résidu d'acide aminé a été remplacé par un autre acide aminé ayant un index hydropathique similaire.
Par inhibiteur de métalloprotéinase, on entend toute molécule capable de réguler l'activité des MMPs soit au niveau de l'expression des gènes (transcription et traduction), soit au niveau de l'activation de la forme zymogène des MMPs, soit encore au niveau du contrôle local des formes actives.

Les principaux régulateurs de l'activité des MMPs sont des molécules naturelles présentes dans les tissus appelées inhibiteurs tissulaires des métalloprotéinases ou TIMPs (tissue inhibitors of metalloproteinases).

Selon l'invention, on utilise des inhibiteurs tissulaires de métalloprotéinases (TIMP) comme par exemple les peptides connus dans l'art antérieur sous les appellations TIMP-1, TIMP-2, TIMP-3 et TIMP-4 (Woessner J. F., Faseb Journal, 1991).

Ainsi, l'invention concerne plus particulièrement l'utilisation dans/ou pour la préparation d'une composition, d'une quantité efficace d'au moins un inhibiteur tissulaire de métalloprotéinases (TIMP) ou de tout équivalent biologique fonctionnel, l'inhibiteur ou la composition étant destinés à induire et/ou stimuler la croissance des cheveux ou des poils et/ou freiner leur chute.

Particulièrement selon l'invention, on utilise comme inhibiteurs tissulaires de métalloprotéinases les peptides connus dans l'art antérieur sous les appellations TIMP-1, TIMP-2, TIMP-3 et TIMP-4.

Bien entendu selon l'invention, les inhibiteurs de métalloprotéinases peuvent être utilisés seuls ou en mélange.

Il se peut que pour des questions de résistance à la dégradation il soit nécessaire d'utiliser selon l'invention une forme protégée de l'inhibiteur de métalloprotéinases. La forme de la protection doit évidemment être une forme biologiquement compatible. De nombreuses formes de protections biologiquement compatibles peuvent être envisagées comme par exemple l'acylation ou l'acétylation de l'extrémité amino-terminale ou l'amidation de l'extrémité carboxy-terminale.

Ainsi, l'invention concerne une utilisation telle que précédemment définie, caractérisée par le fait que l'inhibiteur de métalloprotéinases est sous une forme protégée ou non.
De préférence, on utilise selon l'invention une protection basée soit sur l'acylation ou l'acétylation de l'extrémité amino-terminale, soit sur l'amidation de l'extrémité carboxy-terminale soit encore sur les deux.

La quantité d'inhibiteur de métalloprotéinases utilisable selon l'invention dépend bien évidemment de l'effet recherché et doit être en une quantité efficace pour induire et/ou stimuler la croissance des cheveux ou des poils et/ou freiner leur chute.

A titre d'exemple la quantité d'inhibiteur de métalloprotéinases utilisable selon l'invention peut aller par exemple de 0,01% à 5% et de préférence de 0,05% à 2% du poids total de la composition.

De préférence la composition est une composition cosmétique.

La composition selon l'invention peut être administrée par voie entérale ou parentérale. De préférence, la composition est administrée par voie topique.

Le milieu physiologiquement acceptable dans lequel le peptide est utilisé selon l'invention peut être anhydre ou aqueux. On entend par milieu anhydre, un milieu solvant contenant moins de 1% d'eau. Ce milieu peut être constitué d'un solvant ou d'un mélange de solvants choisi plus particulièrement parmi les alcools inférieurs en C₂-C₄ comme l'alcool éthylique, les alkylènegtycols comme le propylèneglycol, et les alkyléthers d'alkylèneglycols ou de dialkylèneglycols, dont les radicaux alkyle ou alkylène contiennent de 1 à 4 atomes de carbone. On entend par milieu aqueux, un milieu constitué par de l'eau ou un mélange d'eau et d'un autre solvant physiologiquement acceptable, choisi notamment parmi les solvants organiques cités ci-dessus. Dans ce dernier cas, ces autres solvants, lorsqu'ils sont présents, représentent environ 5 à 95% en poids de la composition.

Il est possible que le milieu physiologiquement acceptable puisse contenir d'autres adjuvants habituellement utilisés dans le domaine cosmétique tels que des agents tensioactifs, des agents épaississants ou gélifiants, des agents cosmétiques, des agents conservateurs, des agents alcalinisants ou acidifiants bien connus·dans l'état de la technique, et en quantités suffisantes pour obtenir la forme de présentation désirée, notamment de lotion plus ou moins épaissie, de gel, d'émulsion, ou de crème. L'utilisation peut éventuellement se faire sous une forme pressurisée en aérosol ou vaporisée à partir d'un flacon pompe.

Il est possible aussi d'utiliser en association avec le peptide, des composés améliorant encore l'activité sur la repousse et/ou sur le freinage de la chute des cheveux ou des poils, et ayant déjà été décrits pour cette activité.

Parmi ces derniers composés, on peut plus particulièrement citer à titre non limitatif :
- les esters d'acide nicotinique, dont notamment le nicotinate de tocophérol, le nicotinate de benzyle et les nicotinates d'alkyles en C₁-C₆ comme les nicotinates de méthyle ou d'hexyle ;
- les dérivés de pyrimidine, comme la 6-amino-1,2-dihydro-1-hydroxy-2-imino-4-pipéridinopyrimidine encore connu sous le nom de Minoxidil, et tels que décrits dans le brevet US 4 139 619 ;
- des agents antiandrogènes ;
- des inhibiteurs des 5-α-réductases;
- des capteurs de radicaux OH, tels que le diméthylsulfoxyde ;
- des peptides comme par exemple le tripeptide Lys-Pro-Val ;
- des extraits de micro-organismes, particulièrement bactériens ;
- des extraits végétaux.

A la liste ci-dessus, d'autres composés peuvent également être rajoutés, à savoir par exemple des phospholipides comme la lécithine, les acides linoléique et linolénique, l'acide salicylique et ses dérivés décrits dans le brevet français FR 2 581 542, comme les dérivés de l'acide salicylique porteurs d'un groupement alcanoyle ayant de 2 à 12 atomes de carbone en position 5 du cycle benzénique, des acides hydroxycarboxyliques ou cétocarboxyliques et leurs esters, des lactones et leurs sels correspondants, des caroténoides, les acides eicosatétraénoïque et eicosatriénoïque ou leurs esters et amides, la vitamine D et ses dérivés.

La composition cosmétique selon l'invention est à appliquer sur les zones alopéciques du cuir chevelu et des cheveux ou des poils d'un individu, et est éventuellement laissée en contact plusieurs heures et est éventuellement rincée. On peut, par exemple, appliquer la composition cosmétique contenant une quantité efficace d'au moins un inhibiteur tissulaire de métalloprotéinases sur les cheveux et le cuir chevelu, le soir, garder celle-ci au contact toute la nuit et éventuellement effectuer un shampooing le matin. Ces applications peuvent être renouvelées quotidiennement pendant un ou plusieurs mois suivant les individus.

Ainsi, la présente invention à également pour objet un procédé de traitement cosmétique des cheveux ou des poils et/ou du cuir chevelu, caractérisé par le fait qu'il consiste à appliquer sur les cheveux et/ou le cuir chevelu, une composition cosmétique contenant une quantité efficace d'au moins un inhibiteur tissulaire de métalloprotéinases, à laisser celle-ci en contact avec les cheveux et/ou le cuir chevelu et/ou les poils, et éventuellement à rincer.

Le procédé de traitement présente les caractéristiques d'un procédé cosmétique dans la mesure où il permet d'améliorer l'esthétique des cheveux ou des poils en leur donnant une plus grande vigueur et un aspect amélioré.

On va maintenant donner à titre d'illustration des exemples qui ne sauraient limiter en aucune façon la portée de l'invention.

| Exemple 1 : Lotion quotidienne : | | | |
|---|---|---|---|
| TIMP-1 | | 0,01 | g |
| TIMP-2 | | 0.01 | g |
| 2,4-diaminopyrimidine-3-oxyde | | 0,75 | g |
| Ethanol à 95° | | 30 | g |
| Parfum | | qs | |
| Colorants | | qs | |
| Eau déminéralisée | qsp | 100 | g |

| Exemple 2 : Gel Liposomé : | | |
|---|---|---|
| Natipide II① (soit 2 g en phospholipides) | 10 | g |
| TIMP-2 | 0,025 | g |
| Carbomer | 0,25 | g |
| Triéthanolamine | qs | pH = 7 |
| Conservateurs | qs | |
| Eau déminéralisée qsp | 100 | g |

| | | |
|---|---|---|
| ①Mélange Eau/Alcool/Lécithine de la Société Nattermann | | |

## Revendications

1. Utilisation dans et/ou pour la préparation d'une composition, d'une quantité efficace d'au moins un inhibiteur de métalloprotéinases ou de tout équivalent biologique fonctionnel, l'inhibiteur ou la composition étant destinés à induire et/ou stimuler la croissance des cheveux ou des poils et/ou freiner leur chute, l'inhibiteur de métalloprotéinase étant un inhibiteur tissulaire de métalloprotéinases.

2. Utilisation selon la revendication 1, **caractérisée par le fait que** l'inhibiteur tissulaire de métalloprotéinases est choisi parmi le TIMP1, le TIMP2, le TIMP-3 ou le TIMP-4.

3. Utilisation selon l'une quelconque des revendications précédentes, **caractérisée par le fait que** l'inhibiteur tissulaire de métalloprotéinases est un peptide d'origine naturelle ou synthétique.

4. Utilisation selon l'une quelconque des revendications précédentes, **caractérisée par le fait que** l'inhibiteur tissulaire de métalloprotéinases est sous une forme protégée ou non.

5. Utilisation selon la revendication précédente, **caractérisée par le fait que** la protection consiste en une protection basée soit sur l'acylation ou l'acétylation de l'extrémité amino-terminale, soit sur l'amidation de l'extrémité carboxy-terminale soit encore sur les deux.

6. Utilisation selon l'une quelconque des revendications précédentes, **caractérisée par le fait que** l'inhibiteur tissulaire de métalloprotéinases est le TIMP-1 ou le TIMP-2.

7. Utilisation selon l'une quelconque des revendications précédentes, **caractérisée par le fait que** l'inhibiteur de métalloprotéinases est utilisé en une quantité comprise entre 0,01% à 5% et de préférence de 0,05% à 2% du poids total de la composition.

8. Utilisation selon l'une quelconque des revendications précédentes, **caractérisée en ce que** la composition contient en outre un composé améliorant la repousse et/ou freinant la chute des cheveux ou des poils choisis parmi les esters d'acide nicotinique, dont notamment le nicotinate de tocophérol, le nicotinate de benzyle et les nicotinates d'alkyles en C₁-C₆ comme les nicotinates de méthyle ou d'hexyle ; les dérivés de pyrimidine, comme le 6-amino-1,2-dihydro-1-hydroxy-2-imino-4-pipéridinopyrimidine encore connu sous le nom de Minoxidil, des agents antiandrogènes ; des inhibiteurs des 5-α-réductases ; des capteurs de radicaux OH, tels que le diméthylsulfoxyde ; des peptides comme par exemple le tripeptide Lys-Pro-Val ; des extraits de micro-organismes, particulièrement bactériens et des extraits végétaux.

9. Procédé de traitement cosmétique des cheveux ou des poils et/ou du cuir chevelu, **caractérisé par le fait qu'**il consiste à appliquer sur les cheveux et/ou le cuir chevelu, une composition contenant une quantité efficace d'au moins un inhibiteur de métalloprotéinases tel que défini dans l'une des revendications 1 à 8, à laisser celle-ci en contact avec les cheveux et/ou le cuir chevelu, et éventuellement à rincer.

## Patentansprüche

1. Verwendung einer wirksamen Menge mindestens eines Metall-Proteinase-Inhibitors oder beliebiger, in ihrer biologischen Funktion äquivalenter Verbindungen in einer Zusammensetzung und/oder zur Herstellung einer Zusammensetzung, wobei der Inhibitor oder die Zusammensetzung dazu vorgesehen sind, das Wachstum der Haare oder der Körperhaare zu induzieren und/oder zu stimulieren und/ oder den Haarausfall zu verlangsamen, wobei es sich bei dem Metall-Proteinase-Inhibitor um einen Metall-Proteinase-Gewebsinhibitor handelt.

2. Verwendung nach Anspruch 1, **dadurch gekennzeichnet, dass** der Metall-Proteinase-Gewebsinhibitor unter TIMP-1, TIMP-2, TIMP-3 und TIMP-4 ausgewählt ist.

3. Verwendung nach einem der vorhergehenden Ansprüche, **dadurch gekennzeichnet, dass** der Metall-Proteinase-Gewebsinhibitor ein natürliches oder synthetisches Peptid ist.

4. Verwendung nach einem der vorhergehenden Ansprüche, **dadurch gekennzeichnet, dass** der Metall-Proteinase-Gewebsinhibitor in geschützter oder ungeschützter Form vorliegt.

5. Verwendung nach dem vorhergehenden Anspruch, **dadurch gekennzeichnet, dass** der Schutz entweder auf Acylierung oder Acetylierung der endständigen Aminogruppe oder Amidierung der endständigen Carboxygruppe oder auf beiden Maßnahmen basiert.

6. Verwendung nach einem der vorhergehenden Ansprüche, **dadurch gekennzeichnet, dass** der Metall-Proteinase-Gewebsinhibitor unter TIMP-1 oder TIMP-2 ausgewählt ist.

7. Verwendung nach einem der vorhergehenden Ansprüche, **dadurch gekennzeichnet, dass** der Metall-Proteinase-Gewebsinhibitor in einer Menge von 0,01 bis 5 % und vorzugsweise 0,05 bis 2 % des Gesamtgewichts der Zusammensetzung verwendet wird.

8. Verwendung nach einem der vorhergehenden Ansprüche, **dadurch gekennzeichnet, dass** die Zusammensetzung ferner einer Verbindung enthält, die den Haarwuchs fördert und/oder das Ausfallen der Haare oder Körperhaare verlangsamt und unter Nicotinsäureestern, insbesondere Tocopherylnicotinat, Benzylnicotinat und C₁₋₆-Alkylnicotinaten, wie Methylnicotinat oder Hexylnicotinat; Pyrimidinderivaten, wie 6-Amino-1,2-dihydro-1-hydroxy-2-imino-4-piperidino-pyrimidin, das auch unter der Bezeichnung Minoxidil bekannt ist; Antiandrogenen; 5α-Reductasehemmern; Radikalfängern für OH-Radikale, wie Dimethylsulfoxid; Peptiden, wie beispielsweise dem Tripeptid Lys-Pro-Val; Extrakten von Mikroorganismen, insbesondere Bakterienextrakten; und Pflanzenextrakten ausgewählt ist.

9. Verfahren zur kosmetischen Behandlung der Haare, der Körperhaare und/oder der Kopfhaut, **dadurch gekennzeichnet, dass** auf das Haar und/oder die Kopfhaut eine kosmetische Zusammensetzung nach einem der Ansprüche 1 bis 8 aufgetragen wird, die mindestens einen oben definierten Metall-Proteinase-Inhibitor enthält, das Haar und/oder die Kopfhaut mit der Zusammensetzung in Kontakt zu belassen und ggf. zu spülen.

## Claims

1. Use, in and/or for the preparation of a composition, of an effective amount of at least one metalloprotease inhibitor or of any functional biological equivalent, the inhibitor or composition being intended to induce and/or stimulate the growth of head hair or other hairs and/or to slow down their loss, the metalloprotease inhibitor being a tissue inhibitor of metalloproteases.

2. Use according to Claim 1, **characterized in that** the tissue inhibitor of metalloproteases is chosen from TIMP-1, TIMP-2, TIMP-3 and TIMP-4.

3. Use according to either of the preceding claims, **characterized in that** the tissue inhibitor of metalloproteases is a peptide of natural or synthetic origin.

4. Use according to any one of the preceding claims, **characterized in that** the tissue inhibitor of metalloproteases is in a protected or unprotected form.

5. Use according to the preceding claim, **characterized in that** the protection consists of a protection based either on the acylation or acetylation of the amino-terminal end, or on the amidation of the carboxy-terminal end, or alternatively on both approaches.

6. Use according to any one of the preceding claims, **characterized in that** the tissue inhibitor of metalloproteases is TIMP-1 or TIMP-2.

7. Use according to any one of the preceding claims, **characterized in that** the metalloprotease inhibitor is used in an amount of between 0.01% and 5% and preferably from 0.05% to 2% relative to the total weight of the composition.

8. Use according to any one of the preceding claims, **characterized in that** the composition further contains a compound improving the regrowth of head hair or other hairs and/or slowing down their loss which is chosen from nicotinic acid esters, in particular including tocopheryl nicotinate, benzyl nicotinate and C₁-C₆ alkyl nicotinates such as methyl or hexyl nicotinate; pyrimidine derivatives, such as 6-amino-1,2-dihydro-1-hydroxy-2-imino-4-piperidinopyrimidine also known as "Minoxidil"; antiandrogenic agents; 5-α-reductase inhibitors; OH-radical scavengers, such as dimethyl sulphoxide; peptides such as, for example, the tripeptide Lys-Pro-Val; microorganism extracts, particularly bacterial extracts, and plant extracts.

9. Cosmetic treatment process for head hair or other hairs and/or for the scalp, **characterized in that** it consists in applying a composition containing an effective amount of at least one metalloprotease inhibitor as defined in one of Claims 1 to 8 to the hair and/or the scalp, in leaving this composition in contact with the hair and/or the scalp, and optionally in rinsing it off.
